# EUROPEAN PATENT APPLICATION

(11) **EP 4 574 050 A1**
(43) Date of publication of application: **25.06.2025**
(21) Application number: 23855000.8
(22) Date of filing: 14.06.2023
(51) Int. Cl.: A61B 6/51, A61B 6/00, A61B 6/03, A61B 5/00, G16H 50/20, G06N 3/045

(54) **DENTAL LESION INFORMATION VISUALIZATION METHOD AND SYSTEM**

(30) Priority: 18.08.2022 KR 20220103140
(71) Applicant: Osstem Implant Co., Ltd., Gangseo-gu Seoul 07789 (KR)
(72) Inventor: KIM, Jong Moon, Gunpo-si Gyeonggi-do 15827 (KR)
(74) Representative: Klunker IP Patentanwälte PartG mbB
(86) International application number: PCT/KR2023/008157
(87) International publication number: WO 2024/039029

(57) **Abstract**

The present disclosure relates to a dental lesion detection method and a system to which the method is applied. A dental lesion information visualization method according to the present disclosure may comprise the steps of: acquiring data of a lesion analysis model which outputs data on the type and location of a lesion included in a panoramic image obtained by capturing an image of the oral cavity of a patient; and inputting a panoramic image into the lesion analysis model to output data on the type and location of a lesion and a lesion detection confidence score.

## Description

### [Technical Field]

The present disclosure relates to a dental lesion information visualization method and a system to which the method is applied. More particularly, the present disclosure relates to a method of identifying a dental lesion by analyzing an image of a patient's oral cavity, and a system to which the method is applied.

### [Background Art]

Lesions that can be diagnosed through dental imaging related to teeth include various types such as dental caries, periodontal disease, cracks, and periapical inflammation. Among these diverse lesions, in order for a dentist to accurately determine the exact location and size of a lesion related to teeth, computed tomography (CT) imaging may be necessary.

However, CT imaging often raises concerns among patients due to the high cost of the procedure and issues with radiation exposure. In addition, detecting lesions in CT images requires the dentist to spend considerable time analyzing the images.

As a result, when a patient visits a dental clinic, a panoramic image is initially taken, and the dentist then visually examines the lesions in the oral cavity by analyzing a panoramic image.

However, since the panoramic image is created by unfolding predetermined arches of the maxilla and mandible onto a flat plane, if a lesion is located in an area overlapping with adjacent structures due to anatomical configurations, it may be challenging for the dentist to visually identify the lesion on the panoramic image. To assist the dentist in reducing the likelihood of failing to visually identify lesions, an artificial intelligence (AI)-based method for visualizing dental lesion information can be considered. However, as mentioned above, if the lesion is located in an overlapping area due to anatomical configurations, the accuracy of AI-based dental lesion visualization may also decrease.

As described above, in certain cases, such as when a lesion is located in an area overlapping with adjacent structures, both visual identification on a panoramic image and AI-based dental lesion visualization may be challenging. Despite this, there is currently no panoramic imaging viewer software or panoramic imaging device available that can clearly inform the dentist whether or not it is a case in which visualization of dental lesion information would be difficult. Consequently, unnecessary CT scans may be performed, leading to waste in terms of time and cost.

### [Disclosure]

### [Technical problem]

An objective to be achieved through some embodiments of the present disclosure is to provide a method of displaying data related to a suspected area of an unclear dental lesion on a panoramic image and a computing system to which the method is applied.

Another objective to be achieved through some embodiments of the present disclosure is to provide a method of providing a panoramic image viewer that provides a user interface for activating a computed tomography (CT) image viewer or CT imaging module for a suspected dental lesion area on a panoramic image analyzed by a machine-learned artificial intelligence (AI) model with minimal intuitive operations, and a computing system to which the method is applied.

Another objective to be achieved through some embodiments of the present disclosure is to provide a method of analyzing a patient's panoramic image with a machine learning-based dental lesion information visualization method in which multiple artificial neural network models collaborate to improve lesion identification accuracy, and a computing system to which the method is applied.

Another objective to be achieved through some embodiments of the present disclosure is to provide a method of preprocessing a panoramic image to perform visualization of the patient's dental lesion information.

Another objective to be achieved through some embodiments of the present disclosure is to provide a method of identifying the severity of a patient's dental lesion by analyzing a panoramic image of the patient's oral cavity.

Another objective to be achieved through some embodiments of the present disclosure is to provide a method of generating and providing a list of lesions identified in the patient's oral cavity by analyzing a panoramic image of the patient's oral cavity.

Another objective to be achieved through some embodiments of the present disclosure is to provide a method of automatically identifying a lesion, located in an overlapping area between anatomical structures in a patient's panoramic image and thus difficult for a dentist to visually assess, using an AI model.

Objectives of the present disclosure are not limited to those mentioned above, and other objectives not stated here should be clearly understood by those of ordinary skill in the art to which the present disclosure pertains from the description below.

### [Technical Solution]

A dental lesion information visualization method according to one embodiment of the present disclosure may include the steps of inputting data on a panoramic image of a patient's oral cavity to a machine-learned lesion analysis model, determining a region of interest of the panoramic image by using data output by the lesion analysis model, and overlaying and displaying an indicator pointing to the region of interest on the panoramic image.

In one embodiment, the step of determining the region of interest may include the steps of determining a verification-required tooth where a lesion detection confidence score is less than or equal to a reference value by using the data output by the lesion analysis model, identifying a region of the verification-required tooth in the panoramic image, and designating a portion of the region of the verification-required tooth as the region of interest.

In one embodiment, the region of the verification-required tooth may include a first subregion, a second subregion, and a third subregion obtained by sequentially dividing the region from a crown toward a root along a direction perpendicular to a tooth axis, and the step of designating the portion of the region of the verification-required tooth as the region of interest may include the step of designating, among the first to third subregions, a subregion corresponding to a lesion type of the verification-required tooth as the region of interest, wherein the lesion type of the verification-required tooth is determined using the data output by the lesion analysis model.

In one embodiment, the step of designating the subregion corresponding to the lesion type of the verification-required tooth as the region of interest may include the step of, when the lesion type of the verification-required tooth is a dental caries type, designating the first subregion as the region of interest, when the lesion type of the verification-required tooth is a periodontitis type, designating the second subregion as the region of interest, and when the lesion type of the verification-required tooth is a periapical lesion type, designating the third subregion as the region of interest.

In one embodiment, the region of the verification-required tooth may include a first subregion, a second subregion, and a third subregion obtained by sequentially dividing the region from a crown toward a root along a direction perpendicular to a tooth axis, the first subregion may include 1-1^{st} to 1-N^{th} subregions (where N is a natural number greater than or equal to 2) obtained by dividing the first subregion into N subregions in a direction parallel to a direction of the tooth axis, the second subregion may include 2-1^{st} to 2-N^{th} subregions (where N is a natural number greater than or equal to 2) obtained by dividing the second subregion into N subregions in the direction parallel to the direction of the tooth axis, the third subregion may include3-1^{st} to 3-N^{th} subregions (where N is a natural number greater than or equal to 2) obtained by dividing the third subregion into N subregions in the direction parallel to the direction of the tooth axis, and the step of designating the portion of the region of the verification-required tooth as the region of interest may include the step of designating one of the 1-1^{st} to 1-N^{th} subregions, 2-1^{st} to 2-N^{th} subregions, and 3-1^{st} to 3-N^{th} subregions as a lesion location of the verification-required tooth using the data output by the lesion analysis model.

In one embodiment, the dental lesion information visualization method may further include the step of displaying a computed tomography (CT) image of the patient's oral cavity in response to user selection input on an indicator.

In one embodiment, the step of displaying the CT image of the oral cavity may include the step of displaying the CT image with its field of view (FoV) initially set based on the position of the region of interest.

In one embodiment, the dental lesion information visualization method may further include the steps of searching for the CT image of the patient's oral cavity on a server in response to user selection input on the indicator, and executing a CT imaging module in response to determining that the CT image of the patient's oral cavity is not present based on the search result.

A dental lesion information visualization method according to another embodiment of the present disclosure may include the steps of: inputting data on a panoramic image of a patient's oral cavity to a machine-learned lesion analysis model, generating a lesion detection list using data output by the lesion analysis model, and displaying the lesion detection list together with the panoramic image.

The lesion detection list may include a tooth identification number of a tooth in which a lesion is identified, the type of detected lesion, a lesion detection confidence score for each identified lesion.

In one embodiment, the dental lesion information visualization method may further include the steps of searching for a CT image of the patient's oral cavity in response to user selection input on a record of a first lesion included in the lesion detection list, and executing a CT imaging module in response to determining that the CT image of the patient's oral cavity is not present based on the search result.

A dental lesion information visualization method according to still another embodiment of the present disclosure may include the steps of: acquiring data of a lesion analysis model that outputs data on a type and location of a lesion included in a panoramic image of a patient's oral cavity, the lesion analysis model comprising a first artificial neural network, a second artificial neural network, and a third artificial neural network, inputting data of the panoramic image to the first artificial neural network and performing segmentation processing on each tooth region included in the panoramic image by using data output from the first artificial neural network, inputting data of the segmentation-processed panoramic image to the second artificial neural network and identifying a lesion included in the panoramic image for each tooth by using data output from the second artificial neural network, and inputting an image of the tooth region with the identified lesion from the segmentation-processed panoramic image to the third artificial neural network and outputting data on the type and location of a lesion and lesion detection confidence score by using data output from the third artificial neural network.

In one embodiment, the step of performing segmentation processing on each tooth region included in the panoramic image may include the steps of cropping the panoramic image to display only a region of interest, masking tooth, crown, tooth pulp, and bone level regions in the cropped panoramic image and inputting the masked panoramic image to the first artificial neural network.

In one embodiment, the step of identifying a lesion included in the panoramic image for each tooth may include the steps of: dividing each tooth region included in the segmentation-processed panoramic image into first through third subregions by sequentially dividing the tooth region from a crown toward a root along a direction perpendicular to a tooth axis; inputting data of the first subregion to the second artificial neural network of a first type and identifying the presence of a dental caries lesion in the panoramic image by using data output from the second artificial neural network of a first type; inputting data of the second subregion to the second artificial neural network of a second type and identifying the presence of a periodontal disease lesion in the panoramic image by using data output from the second artificial neural network of a second type; and inputting data of the third subregion to the second artificial neural network of a third type and identifying the presence of a periapical inflammation lesion in the panoramic image by using data output from the second artificial neural network of a third type.

In one embodiment, the step of identifying the presence of a periodontal disease lesion in the panoramic image may include the step of identifying the presence of a periodontal disease lesion in the panoramic image by using data on an extent of depression of a bone level region relative to a crown region included in the second subregion, the data being output from the second artificial neural network of a second type.

In one embodiment, the step of identifying the presence of a periapical inflammation lesion in the panoramic image may include the step of identifying the presence of a periapical inflammation lesion in the panoramic image by using data indicating an inflammation region in the third subregion, which is adjacent to a tooth and lacks Hounsfield unit (HU) values corresponding to bone and tooth, the data being output from the second artificial neural network of a third type.

In one embodiment, the step of identifying the presence of a dental caries lesion in the panoramic image may include the step of identifying the presence of a dental caries lesion in the panoramic image by using data indicating an inflammation region with an HU value different from that of a crown region included in the first subregion, the data being output from the second artificial neural network of a first type.

A dental lesion information visualization system according to yet another embodiment of the present disclosure may include one or more processors and a memory configured to load one or more instructions. Here, the one or more processors may, by executing the one or more stored instructions, perform operations including: inputting data on a panoramic image of a patient's oral cavity to a machine-learned lesion analysis model; determining a second region of interest in the panoramic image by using data output by the lesion analysis model; overlaying and displaying an indicator pointing to the region of interest on the panoramic image; generating a lesion detection list using the data output by the lesion analysis model; and displaying the lesion detection list together with the panoramic image.

### [Description of Drawings]

FIG. 1 illustrates an exemplary environment to which a dental lesion information visualization system according to an embodiment of the present disclosure can be applied.
FIG. 2 is a block diagram illustrating the dental lesion information visualization system described with reference to FIG. 1.
FIG. 3 is a flowchart illustrating a dental lesion information visualization method according to another embodiment of the present disclosure.
FIG. 4 is a flowchart for describing some operations in detail as described with reference to FIG. 3.
FIG. 5 illustrates, by way of example, the result of detecting input on an indicator and displaying an oral computed tomography (CT) image as described with reference to FIG. 3.
FIG. 6 illustrates, by way of example, a subregion corresponding to a lesion type of verification-required tooth for understanding of some embodiments of the present disclosure.
FIG. 7 illustrates subregions of a verification-required tooth for understanding some embodiments of the present disclosure.
FIG. 8 illustrates an example screen in which a region of interest is overlaid and displayed on a panoramic image according to some embodiments of the present disclosure.
FIG. 9 is a flowchart illustrating a dental lesion information visualization method according to yet another embodiment of the present disclosure.
FIG. 10 illustrates, by way of example, the result of performing the step of generating a lesion detection list as shown in FIG. 9.
FIG. 11 illustrates, by way of example, the result of performing the step of detecting input on the lesion record and displaying an oral CT image as shown in FIG. 9.
FIG. 12 illustrates the result of performing the step of displaying data output by a lesion analysis model on a user terminal in conjunction with a patient management system, as may be performed in some embodiments of the present disclosure.
FIG. 13 is a flowchart illustrating a dental lesion information visualization method according to still another embodiment of the present disclosure.
FIGS. 14 and 15 illustrate, by way of example, the results of preprocessing a panoramic image, as may be performed in some embodiments of the present disclosure.
FIGS. 16 to 18 illustrate, by way of example, the results of segmentation preprocessing of each anatomical structure included in a panoramic image, as may be performed in some embodiments of the present disclosure.
FIGS. 19 to 23 are diagrams illustrating, by way of example, a method for identifying a lesion included in a panoramic image, as may be performed in some embodiments of the present disclosure.
FIG. 24 illustrates a hardware configuration of a dental lesion information visualization system according to an embodiment of the present disclosure.

### [Mode of the Invention]

Hereinafter, example embodiments of the present disclosure will be described in detail with reference to the accompanying drawings. The advantages and features of the present disclosure and the manner of achieving the advantages and features will become apparent with reference to embodiments described in detail below together with the accompanying drawings. However, the present disclosure may be implemented in many different forms and should not be construed as being limited to the embodiments set forth herein, and the embodiments are provided such that this disclosure will be thorough and complete and will fully convey the scope of the present disclosure to those skilled in the art, and the present disclosure is defined only by the scope of the appended claims.

In the following description of the present disclosure, a detailed description of known functions and configurations incorporated herein will be omitted to avoid making the subject matter of the present invention unclear.

Hereinafter, some embodiments of the present disclosure will be described with reference to the accompanying drawings.

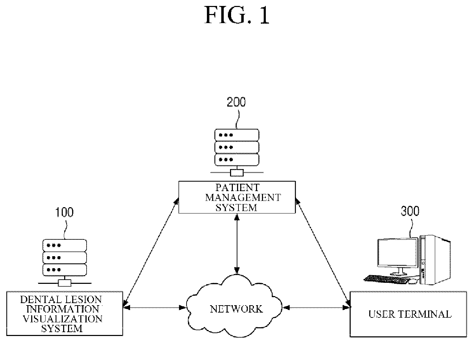

FIG. 1 illustrates an environment to which a dental lesion information visualization system according to an embodiment of the present disclosure can be applied. As shown in FIG. 1, a dental lesion information visualization system 100 according to the present embodiment may implement a dental lesion information visualization method. by interacting with a patient management system 200 and a user terminal 300. Although FIG. 1 illustrates the dental lesion information visualization system 100 and the patient management system 200 as separate systems, in some embodiments, the dental lesion information visualization system 100 and the patient management system 200 may also be implemented as software modules within a single system.

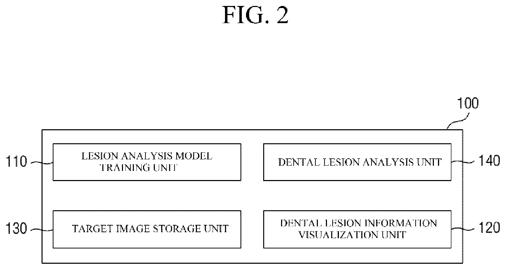

Hereinafter, each component shown in FIGS. 1 and 2 will be described in more detail.

The patient management system 200 is a system that includes patient information and patient images. The patient management system 200 may output the patient's electronic chart according to input from the user terminal 300. Here, the electronic chart may include the patient's personal information, disease history, diagnostic information, and panoramic images of the patient's affected area. Such documents may be medical imaging files (Digital Imaging and Communications in Medicine: DICOM), web documents in formats such as HyperText Markup Language (HTML) or eXtensible Markup Language (XML) that contain the patient's personal information, past test information, and details of the image itself, but are not limited thereto.

Additionally, according to one embodiment of the present disclosure, the patient management system 200 may receive data related to the identified lesion type, detailed location where the lesion was identified, and lesion detection confidence score from the dental lesion information visualization system 100 and display it on the user terminal 300.

The user terminal 300 may output the received electronic chart on a screen. The user terminal 300 may be equipped with a specific display device to present the electronic chart.

Also, the user terminal 300 may also display on the display device a panoramic image with a region of interest set for a dental lesion received from the dental lesion information visualization system 100, along with a CT image corresponding to the region of interest. In addition, it may receive input from the user instructing it to display the panoramic image or CT image.

A target image storage unit 130 of the dental lesion information visualization system 100 may receive data on a panoramic image of the patient's oral cavity and may input the received panoramic image into a lesion analysis model. Here, the panoramic image may also be received from the patient management system 200 in response to a request from the user terminal 300.

According to some embodiments of the present disclosure, a dental lesion analysis unit 140 may analyze a lesion using the patient's panoramic image stored in the target image storage unit 130. The dental lesion analysis unit 140 may transmit the results of analyzing the panoramic image to a lesion analysis model training unit 110. The lesion analysis model training unit 110 may update the lesion analysis model by further training it with the analysis result sent from the dental lesion analysis unit 140 and user feedback.

The dental lesion information visualization system 100 may designate a region of interest in the panoramic image using the data output by the lesion analysis model. The region of interest may refer to an area in the panoramic image that may potentially contain a lesion.

The dental lesion information visualization unit 120 in the dental lesion information visualization system 100 may overlay an indicator pointing to the region of interest on the panoramic image and display it to the user via the display device on the user terminal 300. Additionally, in response to a user selection input on the indicator, the dental lesion information visualization system 100 may display a CT image of the patient's oral cavity, corresponding to the panoramic image, on the user terminal 300. Consequently, the dental lesion information visualization system 100 may intuitively indicate to the user that the indicator is a selectable graphic object, for example, by automatically changing a cursor to a selectable shape in response to a mouse-over event on the indicator.

The operations of determining the region of interest, displaying the indicator to the user, and displaying the CT image may be performed by the dental lesion information visualization unit 120 shown in FIG. 2. More detailed embodiments of these operations will be described further below.

The dental lesion analysis unit 140 in the dental lesion information visualization system 100 may generate a lesion detection list for the lesions included in the input panoramic image. According to some embodiments of the present disclosure, the lesion detection list may be displayed to the user via the display device on the user terminal 300 and may also be stored in the patient management system 200.

Additionally, the dental lesion information visualization unit 120 of the dental lesion information visualization system 100 may receive user input from the user terminal 300 regarding a specific lesion record included in the lesion detection list. **In** response to the input, the dental lesion information visualization system 100 may display an oral CT image of the patient associated with the lesion detection list on the user terminal 300.

The dental lesion information visualization system 100 may preprocess the panoramic image, input the information on the panoramic image to the lesion analysis model to identify a lesion contained in the panoramic image, and output the type and location of the lesion and a lesion detection confidence score. Here, the lesion analysis model includes first to third artificial neural networks, and the dental lesion information visualization system 100 may perform operations described above, on the basis of data output from the multiple neural networks.

The operations of preprocessing the panoramic image, identifying a lesion contained in the panoramic image, and outputting the type, location, and detection confidence score of the lesion may be performed by the dental lesion analysis unit 140 shown in FIG. 2.

Thus far, the configuration and operation of the dental lesion information visualization system 100 and the environment containing the dental lesion information visualization system 100 have been described with reference to FIGS. 1 and 2. The dental lesion information visualization system 100 may be understood to operate according to a client-server model with the user terminal 300. However, in some embodiments, the system may be configured in a client stand-alone manner without requiring a server. In this case, the operations performed by the dental lesion information visualization system 100 described above would be understood to be carried out on the user terminal 300.

According to the present embodiment, a lesion included in an overlapping area between anatomical structures in a patient's panoramic image, which is difficult for a dentist to assess visually, may be automatically identified through an AI model.

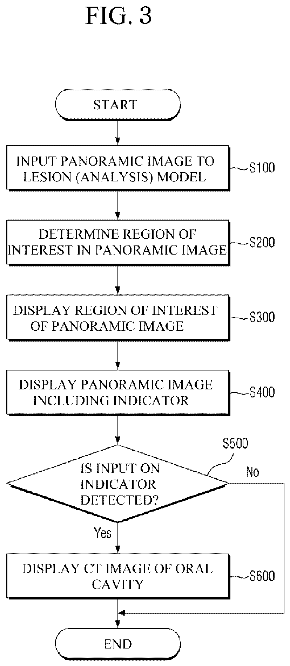

Hereinafter, with reference to FIGS. 3 to 8, a more detailed description of a dental lesion information visualization method according to another embodiment of the present disclosure will be provided. Unless otherwise specified, the steps described in the following flowcharts can be understood to be performed by the dental lesion information visualization system 100.

FIG. 3 is a flowchart illustrating a dental lesion information visualization method according to the present embodiment.

In step S100, the dental lesion information visualization system 100 may input data for a panoramic image of the patient's oral cavity to a lesion analysis model. The panoramic image may be one in which each tooth included in the image has been assigned a tooth identification number.

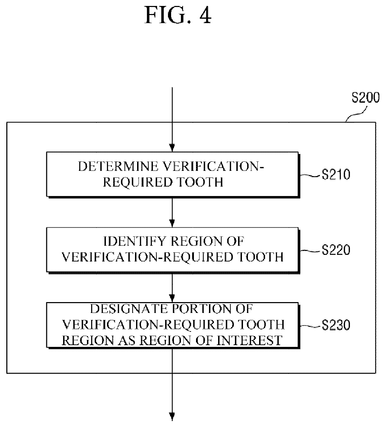

Next, in step S200, the dental lesion information visualization system 100 may designate a region of interest in the panoramic image using data output by the lesion analysis model. A more detailed description of the step of determining the region of interest will be provided with reference to FIGS. 4 to 8.

Referring to FIG. 4, in step S210, the dental lesion information visualization system 100 may determine a verification-required tooth. Here, the verification-required tooth may refer to a tooth for which the confidence score of the lesion analysis model's identification of a lesion within each tooth in the panoramic image is less than or equal to a reference value. In addition, the reference value may be a value preset by a user or an initial setting value of the system.

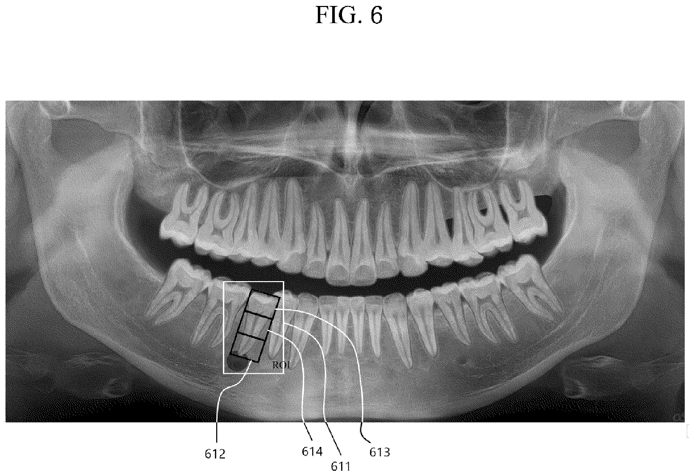

In step S220, the dental lesion information visualization system 100 may identify a region of the verification-required tooth. In FIG. 6, an exemplary region 611 of a verification-required tooth is shown in a box format. Furthermore, the region 611 of the verification-required tooth may be divided sequentially into three sections from the crown toward the root along a direction perpendicular to the tooth axis. Referring to FIG. 6, the three-section divided region 611 of the verification-required tooth includes the uppermost region in the crown direction as a first subregion 613, the region between the first subregion 613 and a third subregion 612 as a second subregion 614, and the lowermost region in the root direction as the third subregion 612.

In some embodiments related to step S220, the verification-required tooth region 611 may be divided sequentially into three sections from the root toward the crown along the direction perpendicular to the tooth axis. Referring to FIG. 6, the three-section divided verification-required tooth region 611 may include the lowermost region in the root direction as a first subregion 612, the region between the first subregion 612 and a third subregion 613 as a second subregion 614, and the uppermost region in the crown direction as a third subregion 613.

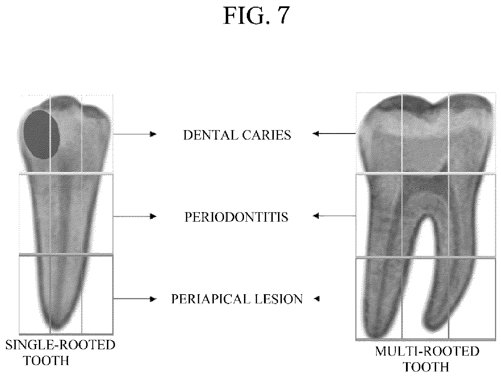

Moreover, each of the first to third subregions may include further subdivided areas. For example, the first subregion may be divided in a direction parallel to the tooth axis direction, comprising subregions from 1-1^{st} to 1-N^{th} subregions. It should be noted that the variable N is a natural number greater than or equal to 2. For example, N may be 3. In other words, as shown in FIG. 7, the region of a verification-required tooth may be divided into a total of 9 (3x3) subregions. Furthermore, these subdivided regions of the verification-required tooth may be referred to as the first, second, and third subregions sequentially from the crown toward the root along the direction perpendicular to the tooth axis. The three regions further divided from the first subregion may be designated, from the mesial to the distal direction, as the 1-1^{st} subregion, 1-2^{nd} subregion, and 1-3^{rd} subregion, respectively.

In step S230, the dental lesion information visualization system 100 may designate a portion of the verification-required tooth region as the region of interest. In FIG. 6, the third subregion 612 is shown as the determined region of interest. The portion of the verification-required tooth region designated as the region of interest may be an area corresponding to the lesion type identified in the lesion analysis for the verification-required tooth.

In some embodiments related to step S230, if data output by the lesion analysis model suggests that the verification-required tooth is likely to have a dental caries-type lesion, the dental lesion visualization system 100 may designate the first subregion as the region of interest. In some embodiments related to step S230, the dental lesion information visualization system 100 may designate the second subregion as the region of interest in response to the data output by the lesion analysis model indicating that the verification-required tooth contains a periodontitis-type lesion.

In yet other embodiments related to step S230, the dental lesion information visualization system 100 may designate the third subregion as the region of interest in response to data output by the lesion analysis model indicating that the verification-required tooth contains a periapical lesion-type lesion.

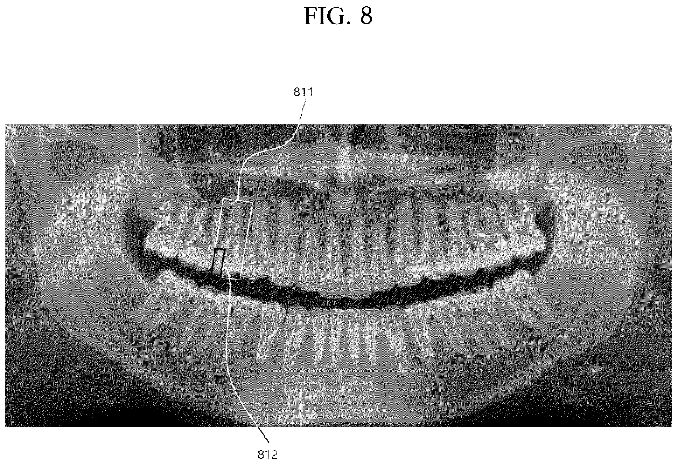

In other embodiments related to step S230, the dental lesion information visualization system 100 may determine the detailed location of the lesion within the designated region of interest based on the data output by the lesion analysis model. To explain the step of determining the lesion location in more detail, reference will be made to FIG. 8.

Referring to FIG. 8, when a verification-required tooth region 811 is designated as a first subregion, the dental lesion information visualization system 100 may designate the 1-3rd subregion 812 located distally within the first subregion as the region of interest containing a dental caries-type lesion based on the data output by the lesion analysis model. Additionally, the 1-3rd subregion 812 may be displayed as an overlay on the patient's panoramic image.

According to the present embodiment, the dental lesion detection system may provide the user with the exact location of a detected dental lesion without requiring user intervention. The identified lesion location using this method may be widely applied in various fields of dental practice, such as dental treatment and dental prosthetic fabrication.

The description will continue with reference to FIG. 3.

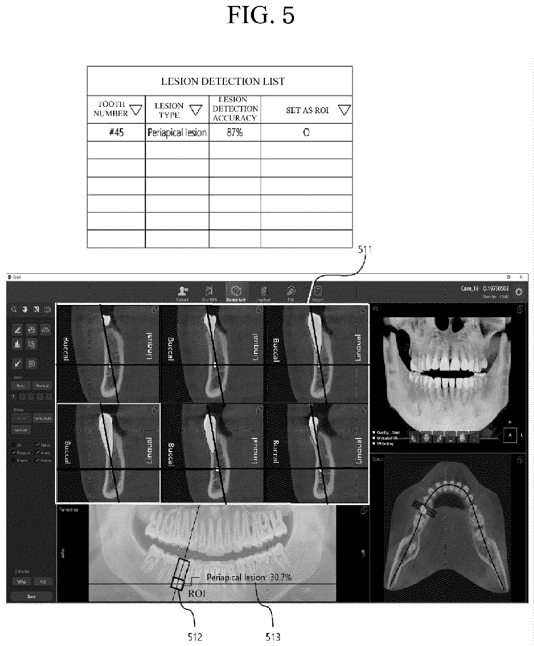

Referring to steps S300 and S400 of FIG. 3 and FIG. 5, in response to the existence of a CT image when the user selects the region of interest on a panoramic view, the dental lesion information visualization system 100 may generate a panoramic view composed by the dental arch of the CT image and display the panoramic image, which includes an indicator 512 of the region of interest, on the user terminal 300. According to some embodiments of the present disclosure, the lesion type and detection confidence score 513 for the lesion within the region of interest may also be displayed.

Next, in step S500, user input related to the indicator on the user terminal 300 may be detected.

In step S600, the dental lesion information visualization system 100 may display a CT cross-sectional image 511 of the patient's oral cavity in response to detecting the user input on the indicator. For example, when the user clicks on the region of interest indicator 512 on the panoramic area displayed on the user terminal, the corresponding CT cross-sectional image 511 of the region of interest is displayed, as shown in FIG. 5. Here, the CT cross-sectional image may be provided aligned with the angle of the tooth axis of the segmented tooth in the region of interest.

In some embodiments related to step S600, the displayed CT image may be shown with the field of view (FoV) initially set based on the position of the region of interest. For example, if the user clicks on the region of interest indicator 512 shown in FIG. 5, the dental lesion information visualization system 100 may display the CT cross-sectional image 511 at an angle that provides the clearest view the lesion in the region of interest.

In some other embodiments related to step S600, in response to the determination that there is no corresponding CT image for the region of interest indicator 612, the dental lesion information visualization system 100 may activate a CT imaging module to acquire a CT image. The CT image acquired through the CT imaging module may be displayed in the same manner as shown in FIG. 5.

According to the present embodiment, the user may view both the CT image and the panoramic image on a single display device, without needing a separate terminal or system to compare the lesion location between the two images. Furthermore, if overlapping anatomical structures in the patient's panoramic image prevent the lesion analysis model from accurately identifying the dental lesion, the system may immediately switch to a user-based diagnostic method.

Thus far, a detailed description has been provided of the dental lesion information visualization method according to one embodiment of this disclosure, where the indicator for a region of interest containing a lesion in a panoramic image is displayed on the user terminal along with the panoramic image. Additionally, it should be noted that each of the steps described so far may be used independently or, if needed, combined in various ways.

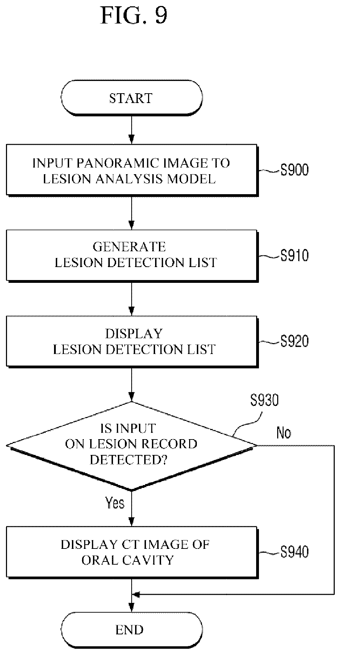

Hereinafter, with reference to FIGS. 9 to 12, a more detailed description of a dental lesion information visualization method according to another embodiment of the present disclosure will be provided.

FIG. 9 is a flowchart illustrating a dental lesion information visualization method according to another embodiment of the present disclosure.

In step S910, the dental lesion information visualization system 100 may generate a lesion detection list based on data output by a lesion analysis model regarding a panoramic image in S900.

The lesion detection list may include a tooth number of a tooth in which a lesion is detected, the name or type of the detected lesion, the confidence score of the lesion detection result, and whether a region of interest is set.

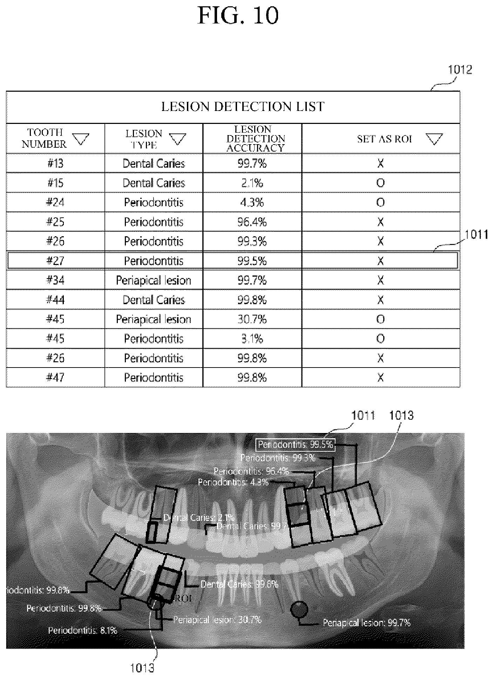

Hereinafter, with reference to FIG. 10, the lesion detection list will be described. In the lesion detection list 1012, for example, a first detection result 1011 may indicate that the region of tooth number 27 in the panoramic image is detected as periodontitis with a probability of 99.5%, and that a region of interest is not set. Here, the step of setting the region of interest may be clearly understood by referring to the detailed description of step S230. When a region of interest is set, an indicator corresponding to the region of interest may appear on the panoramic image, as shown by reference numeral 1013.

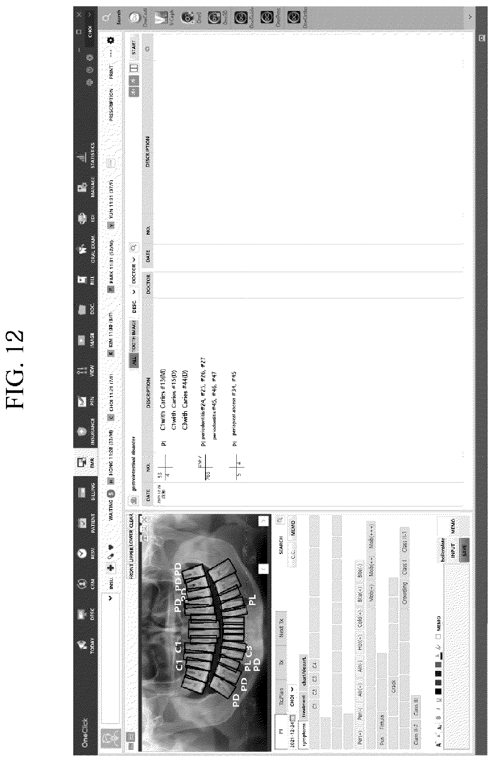

In step S920, referring to FIG. 12, the dental lesion information visualization system 100 may display the lesion detection list and the patient's panoramic image in electronic chart format on the user terminal in conjunction with the patient management system. Here, the display of the lesion detection list in electronic chart format, as shown in FIG. 12, is merely an example provided to aid understanding of the present disclosure, and it should be noted that any method of displaying data output by the lesion detection model may be used without limitation.

In step S930, the dental lesion information visualization system 100 may assess whether user input on the lesion record displayed on the user terminal can be detected. For example, the user input may involve clicking on the area containing lesion name text displayed on the display device of the user terminal, as shown in FIG. 12.

In step S940, the dental lesion information visualization system 100 may display a CT image of the patient's oral cavity on the user terminal in response to detecting the user input on the lesion record displayed on the user terminal.

Referring to FIG. 12, the user terminal may display the type of lesion contained in the lesion record clicked by the user, the tooth number containing the lesion, the lesion detection confidence score, whether a region of interest is set, the location of the detected region of interest in the panoramic image, and a CT image corresponding to the location of the region of interest.

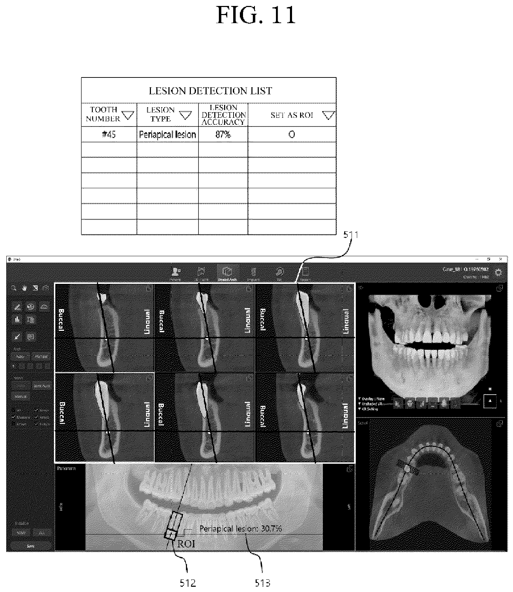

According to the present embodiment, the dental lesion information visualization system 100 allows the user to view both the CT image and panoramic image simultaneously by simply clicking on the displayed detected lesion, as shown in FIG. 11, which may help reduce patient treatment time. Additionally, it is possible to construct a dental arch using tooth axis information obtained by segmenting the CT image and generate it as a panoramic view. The lesion location may then be displayed on the panoramic view corresponding to the tooth number.

Thus far, a detailed description has been provided of the dental lesion information visualization method according to one embodiment of the present disclosure, in which the dental lesion information visualization system 100 displays the CT image on the user terminal in response to user input on the lesion record displayed on the user terminal. Additionally, it should be noted that each of the steps described so far may be used independently or, if needed, combined in various ways.

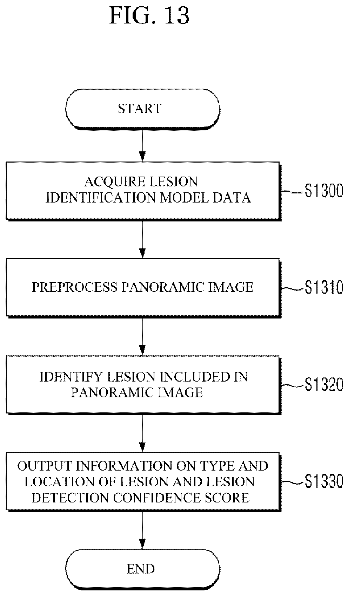

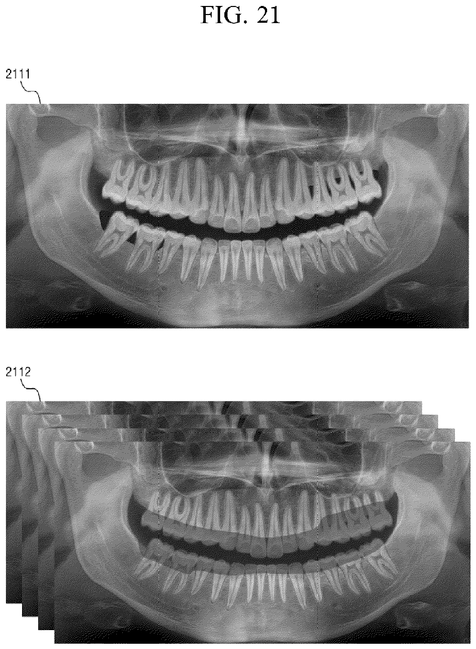

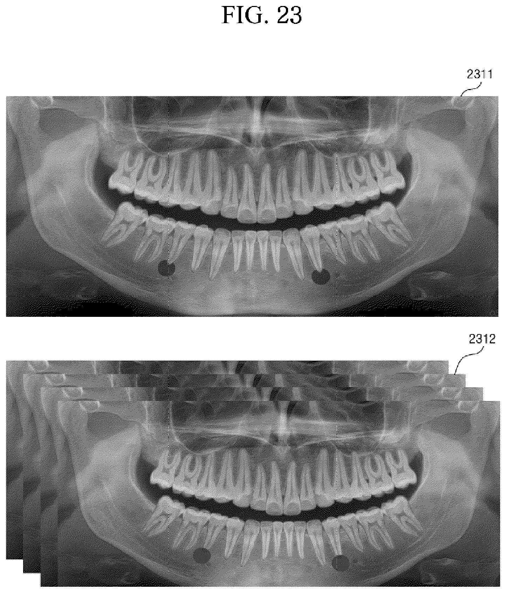

Hereinafter, with reference to FIGS. 13 to 23, a detailed description of a dental lesion information visualization method will be provided according to another embodiment of the present disclosure.

FIG. 13 is a flowchart illustrating a dental lesion information visualization method according to still another embodiment of the present disclosure.

In step S1300, the dental lesion information visualization system 100 may obtain data of a lesion analysis model, trained to output data on the type and location of a lesion contained in a panoramic image of the patient's oral cavity. The lesion analysis model may include first to third artificial neural networks. The data of the lesion analysis model may define each of first to third artificial neural networks, which includes parameters or hyperparameters of each artificial neural network.

Each of the first to third artificial neural networks may be configured using known neural network architectures such as U-NET, convolutional neural networks (CNN), recurrent neural networks (RNN), deep belief networks, or restricted Boltzmann machines.

The first to third artificial neural networks of the lesion analysis model may be trained using data on the panoramic image on which lesion identification has been performed, or data on a panoramic image separately input by the user for neural network training, as well as training data obtained through augmentation of data of these panoramic images. Here, the augmented training data may be generated by inverting the color of the original panoramic image, rotating its angle, flipping the image orientation, or adjusting the window level or window width. In this case, the window level refers to the central value of the grayscale, and the window width may represent the range of HU values that can be represented in grayscale.

The above-described augmentation method for the original training data, i.e., the panoramic images, reflects various cases where dental lesions are further distinguished from normal areas. For example, in the case of a medical image in DICOM format, adjusting the window level and window width may cause a specific lesion to appear or disappear. In addition, applying an invert effect may enhance black-and-white contrast, making lesion detection easier. By comprehensively training on these various modifications of the same panoramic image, the dental lesion information visualization model will achieve high levels of accuracy, sensitivity, and precision.

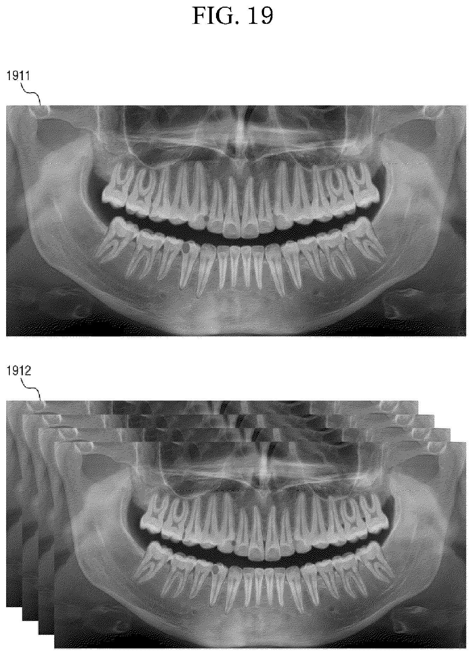

According to some embodiments of the present disclosure, the training data may include dental caries training data 1911, training data 1912 with a masked region of interest for dental caries, periodontal disease training data 2111, training data 2112 with a masked region of interest for periodontal disease, periapical inflammation training data 2311, and training data 2312 with a masked region of interest for periapical inflammation, as shown in FIGS. 19, 21, and 23.

According to this embodiment, the lesion analysis model is generated using augmented training data and masked training data in addition to the original training data, thereby allowing the model to reflect the information contained in the training data from a wide range of perspectives. In the case of a medical image such as a panoramic image, where data is highly scarce, training the lesion analysis model by diversely modifying a limited amount of training data, as described above, may enable the construction of a highly accurate lesion analysis model at a lower cost.

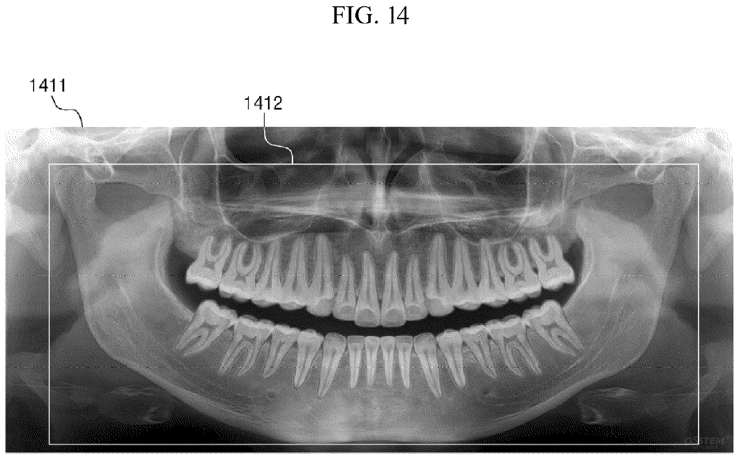

Next, in step S1310, the dental lesion information visualization system 100 may input the panoramic image of the patient, who is the subject of lesion identification, into the first artificial neural network to obtain a panoramic image in which each tooth region is segmented. To explain the step of obtaining the segmentation-processed panoramic image in more detail, reference will be made to FIGS. 14 to 18.

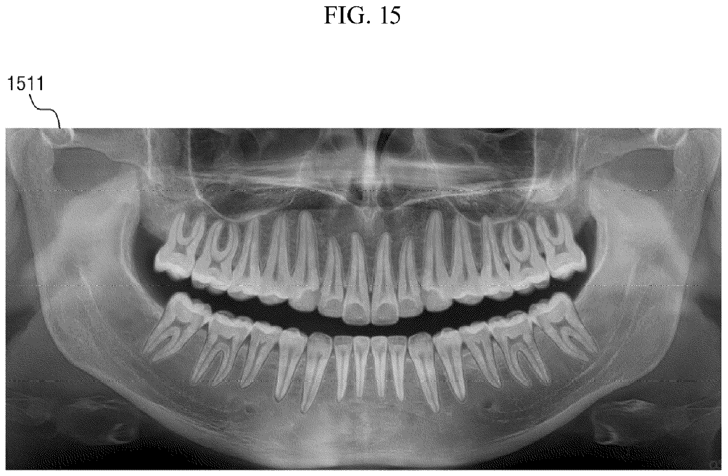

In some embodiments related to step S1310, referring to FIGS. 14 and 15, the dental lesion information visualization system 100 may identify the region containing the tooth region within the panoramic image 1411 as a region of interest 1412. Additionally, a panoramic image 1511 cropped to display only the region of interest may be obtained.

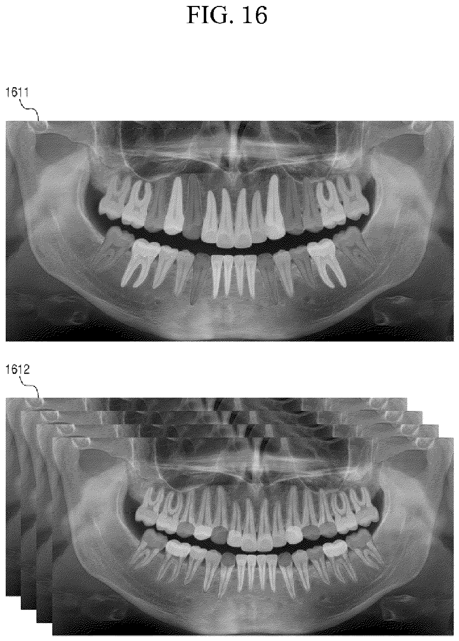

In some other embodiments related to step S1310, referring to reference numeral 1611 in FIG. 16, the dental lesion information visualization system 100 may mask the area corresponding to the tooth in the cropped panoramic image. In addition, as shown by reference numeral 1612, the dental lesion information visualization system 100 may also mask the area corresponding to the tooth crown in the cropped panoramic image.

Here, the action of masking a specific region may refer to adding a distinct marking to ensure the specified area is distinguishable from other regions. In FIGS. 16 to 17 of the present disclosure, marking is represented by assigning a different color to the specific area compared to other regions, but it should be noted that any method that clearly differentiates the specific area from others may be used without limitation.

Additionally, the masking may be performed by executing an image processing algorithm that selects pixels meeting specific criteria or by inputting an image to a machine-learned neural network.

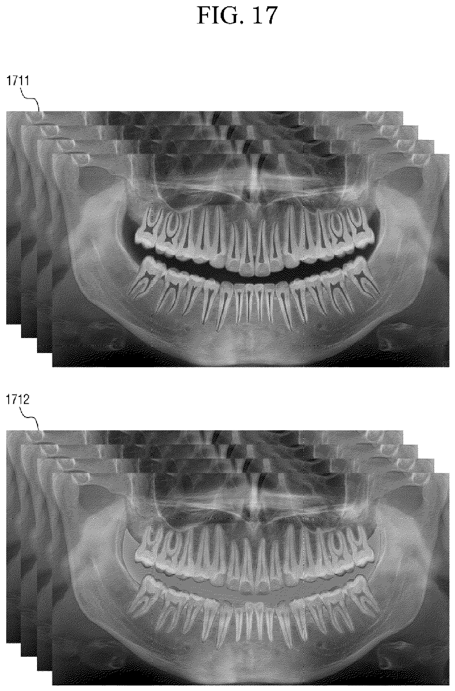

In some other embodiments related to step S1310, referring to reference numeral 1711 of FIG. 17, the dental lesion information visualization system 100 may mask the region corresponding to the tooth pulp in the cropped panoramic image. In addition, referring to a bone level masking result 1712, the dental lesion information visualization system 100 may also mask a bone level area in the cropped panoramic image.

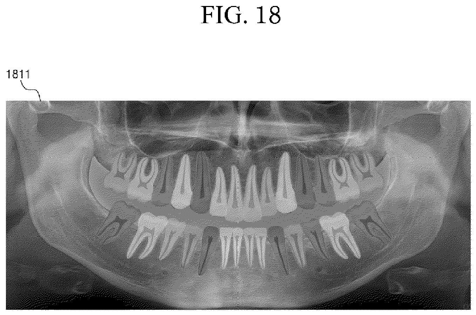

In some other embodiments related to step S1310, referring to FIG. 18, the dental lesion information visualization system 100 may obtain a segmentation-processed panoramic image 1811 in which the masked areas are integrated with distinct markings to make them mutually distinguishable. The segmentation-processed panoramic image 1811 may also be displayed on the display device of the user terminal according to user input.

According to this embodiment, the dental lesion information visualization system 100 may receive the patient's panoramic image and acquire a panoramic image in which regions corresponding to the teeth, crowns, tooth pulp, and bone level are divided and each dental region is assigned a tooth identification number after segmentation processing. The panoramic image with the segmented regions may, of course, be applied to various purposes in the technical field.

Next, in step S1320, the dental lesion information visualization system 100 may input the data from the segmentation-processed panoramic image to the second artificial neural network to identify a lesion contained in each tooth region in the panoramic image.

In some embodiments related to step S1320, the dental lesion information visualization system 100 may divide each tooth region into first through third subregions. More specifically, the dental lesion information visualization system 100 may divide the tooth region into first through third subregions by sequentially dividing the tooth region from the crown toward the root along the direction perpendicular to the tooth axis. The method for dividing the tooth region may be clearly understood by referring to the embodiment related to S220.

In some other embodiments related to step S1320, the dental lesion information visualization system 100 may input data of the first subregion to the second artificial neural network to identify the presence of a dental caries lesion in each tooth region within the panoramic image. Hereinafter, an artificial neural network that receives data of the first subregion and outputs data on the presence of a dental caries lesion will be referred to as a "second artificial neural network of a first type." The data output from the second artificial neural network of a first type on the presence of a dental caries lesion may include information related to the severity of the dental caries lesion.

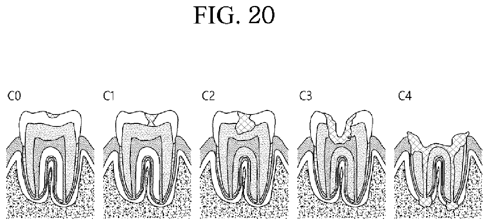

For example, referring to FIG. 20, the data related to the severity of the dental caries lesion may be categorized into at least one stage from C0 to C4 based on the progression of the dental caries lesion. In FIG. 20, C0 may represent an initial cavity form, C1 may be the cavity extending to the enamel layer, C2 may be the cavity reaching the dentin, C3 may represent the cavity progressing to the nerve, and C4 may denote a severe cavity with an abscess formed at the tooth root.

In some other embodiments related to step S1320, if the data output from the second artificial neural network of a first type indicates that a dental caries lesion cannot be identified, it may be due to overlap with adjacent tooth regions, making it impossible to identify the dental caries lesion. To convey data related to this situation, the dental lesion information visualization system 100 may overlay and display an indicator on the panoramic image pointing to the subregion where identification of the dental caries lesion is deemed impossible.

In some other embodiments related to step S1320, the dental lesion information visualization system 100 may input information of the second subregion to the second artificial neural network to identify the presence of a periodontal disease lesion in each tooth region within the panoramic image. Hereinafter, an artificial neural network that receives data of the second subregion and outputs data on the presence of a periodontal disease lesion will be referred to as a "second artificial neural network of a second type."

Here, the second artificial neural network of a second type may output data on the extent of depression of a bone level region relative to the crown region in the segmentation-processed panoramic image. At this point, the dental lesion information visualization system 100 may calculate the extent of depression using the data output from the second artificial neural network of a second type, and if the calculated extent of depression is greater than or equal to a reference value relative to the segmented crown, it may identify that the tooth region containing the second subregion includes a periodontal disease lesion.

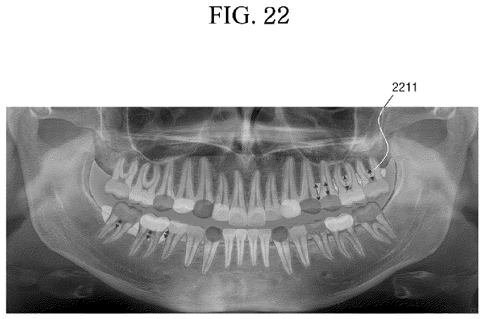

For example, referring to FIG. 22, if a depression distance 2211 of the crown region in the left maxillary second molar region in the panoramic image relative to the bone level region is greater than or equal to the reference value, the dental lesion information visualization system 100 may identify that the left maxillary second molar region in the panoramic image includes a periodontal disease lesion. In addition, the second artificial neural network may output data related to the severity of the periodontal disease lesion based on the depression distance 2211.

In some other embodiments related to step S1320, the dental lesion information visualization system 100 may input information of the third subregion to the second artificial neural network to identify the presence of a periapical inflammation lesion in each tooth region within the panoramic image. Hereinafter, an artificial neural network that receives data of the third subregion and outputs data on the presence of a periapical inflammation lesion will be referred to as a "second artificial neural network of a third type."

The second artificial neural network of a third type may receive data on the third subregion and output data indicating an inflammation region, which is adjacent to a tooth and lacks Hounsfield unit (HU) values corresponding to bone and tooth.

By using the data output from the second artificial neural network of a third type, the dental lesion information visualization system 100 may identify the inflammation region within the third subregion. By using the shape of the inflammation region, the dental lesion information visualization system 100 may determine whether the third subregion includes a periapical inflammation lesion. For example, the dental lesion identification system may measure the roundness of the inflammation region and, if the roundness exceeds a reference value, determine that the third subregion contains a periapical inflammation lesion.

The patient's panoramic image represents the patient's arched oral structure in a flat plane, which may introduce distortion in the image. Therefore, the panoramic image may include some overlapping anatomical structures. Thus, there may be errors in visual diagnoses by the dentist. According to this embodiment, the lesion analysis model preprocesses the patient's panoramic image in a way suitable for identifying dental lesions and determines whether the lesion is identified, based on standard criteria with quantitative values, enabling accurate detection of dental lesions.

In step S1330, the dental lesion identification system inputs the panoramic image of the tooth region containing the identified lesion to the third artificial neural network to obtain data on the lesion's type, location, and detection confidence score. Preferably, the dental lesion information visualization system 100 may use the obtained information to generate a lesion detection list and display the lesion detection list on the display device of the user terminal. The method of displaying the lesion detection list will be clearly understood by referring to the description of some embodiments of step S920 above.

Furthermore, the dental lesion information visualization system 100 may use the obtained data on the lesion location to mark the specific location containing the lesion in the patient's panoramic image as the region of interest and may display it on the display device of the user terminal. The method of displaying the data on the lesion location will be clearly understood by referring to some embodiments of step S400 described above with reference to FIG. 5.

As described above, the first to third artificial neural networks sequentially perform the processing of tooth segmentation and analysis of the presence of dental caries lesions, periodontal disease lesions, and periapical inflammation lesions in each tooth region, thereby providing high accuracy in detecting dental lesions. In other words, to achieve the ultimate goal of dental lesion information visualization, the described embodiments provide an optimal network architecture by sequentially linking the first, second, and third neural networks.

Moreover, through the sequential linking of the first, second, and third neural networks, the lesion type, lesion location, and lesion detection confidence score for a tooth region containing a periodontal disease lesion may all be output.

Additionally, the computing system enhances the visibility of information by displaying the panoramic image together with the tooth region containing a periodontal disease lesion and also lesion type information, allowing the dentist to easily recognize data related to dental lesion visualization.

Moreover, the computing system provides data on a lesion location where the lesion detection confidence score is less than or equal to the reference value and thus a CT image review is necessary. When the user selection input occurs, such as clicking on the lesion location, the computing system may display a CT image with an FoV setting that provides an optimal view of the lesion, or activate the CT imaging module, thereby guiding the user to review or capture a CT image for a region of interest where lesion identification is unclear. Thus, it may be possible to reduce the likelihood that the dentist will misidentify a lesion based solely on the panoramic image.

A detailed description has been provided thus far regarding the dental lesion information visualization method according to an embodiment of the present disclosure. Additionally, it should be noted that each of the steps described so far may be used independently or, if needed, combined in various ways.

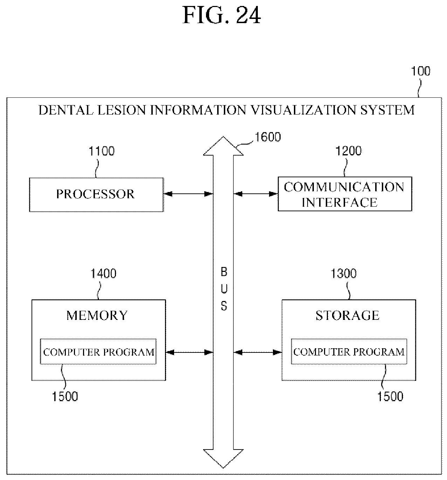

FIG. 24 illustrates the hardware configuration of a dental lesion information visualization system 100 according to some embodiments of the present disclosure. A dental lesion information visualization system 100 shown in FIG. 24 may, for example, refer to the dental lesion information visualization system 100 described with reference to FIG. 1. The dental lesion information visualization system 100 may include at least one processor 1100, a system bus 1600, a communication interface 1200, a memory 1400 configured to load a computer program 1500 executed by the processor 1100, and a storage 1300 configured to store the computer program 1500.

The processor 1100 controls the overall operation of each configuration of the dental lesion information visualization system 100. The processor 1100 may perform computations for at least one application or program for executing methods and/or operations according to various embodiments of the present disclosure. The memory 1400 stores various types of data, commands, and/or information. The memory 1400 may load one or more programs 1500 from the storage 1300 in order to execute the methods and/or operations according to various embodiments of the present disclosure. The bus 1600 provides communication functionality between the components of the dental lesion information visualization system 100. The communication interface 1200 supports Internet communication of the dental lesion information visualization system 100. The storage 1300 may non-temporarily store one or more computer programs 1500. The computer programs 1500 may include one or more instructions implementing the methods and/or operations according to various embodiments of the present disclosure. When the computer program 1500 is loaded on the memory 1400, the processor 1100 may execute the one or more instructions to perform the methods and/or operations according to various embodiments of the present disclosure.

In some embodiments, the dental lesion detection system may be configured using one or more physical servers included in a server farm, based on cloud technologies, such as virtual machines.

The computer program 1500 may include instructions for: inputting data on a panoramic image of the patient's oral cavity to a machine-learned lesion analysis model; determining a region of interest in the panoramic image using data output by the lesion analysis model; overlaying and displaying an indicator pointing to the region of interest on the panoramic image; generating a lesion detection list using the data output by the lesion analysis model; displaying the lesion detection list along with the panoramic image; searching for a CT image of the patient's oral cavity in response to user selection input on the indicator; and executing a CT imaging module in response to determining that a CT image of the patient's oral cavity is not present based on the search result.

So far, various embodiments and effects thereof, in which the technical features of the present disclosure have been implemented, have been described with reference to FIGS. 1 to 24. Effects according to the technical features of the present disclosure are not limited to the above-described effects, and other effects not described will be clearly understood by those skilled in the art from the following description.

The technical features of the present disclosure described above may be implemented as computer readable codes on a computer-readable medium. The computer program recorded on the computer-readable recording medium may be transmitted to another computing device through a network such as the Internet and installed in the other computing device, thereby being used in the other computing device.

Although the operations are shown in a specific order in the drawings, it should not be understood that the operations must be performed in the specific order or sequential order shown, or that all the illustrated operations must be executed to achieve the desired results. In certain circumstances, multitasking and parallel processing may be advantageous. In concluding the detailed description, those skilled in the art will appreciate that many variations and modifications can be made to the exemplary embodiments without substantially departing from the principles of the present disclosure. Therefore, the disclosed exemplary embodiments of the invention are used in a generic and descriptive sense only and not for purposes of limitation. The scope of the present disclosure should be interpreted by the claims below, and any technical features within the scope equivalent to the claims should be interpreted as falling within the scope of the technical features defined by the present disclosure.

## Claims

1. A dental lesion information visualization method performed by a computing system, comprising the steps of:
inputting data on a panoramic image of a patient's oral cavity to a machine-learned lesion analysis model;
determining a region of interest that contains a lesion in the panoramic image by using data output by the lesion analysis model;
searching for a computed tomography (CT) image of the patient's oral cavity in response to user selection input on an indicator;
executing a CT imaging module in response to determining that the CT image of the patient's oral cavity is not present based on the search result; and
overlaying and displaying an indicator pointing to the region of interest on the panoramic image.

2. The dental lesion information visualization method of claim 1, wherein the step of determining the region of interest comprises the steps of:
determining a verification-required tooth where a lesion detection confidence score is less than or equal to a reference value by using the data output by the lesion analysis model;
identifying a region of the verification-required tooth in the panoramic image; and
designating a portion of the region of the verification-required tooth as the region of interest.

3. The dental lesion information visualization method of claim 2, wherein the region of the verification-required tooth comprises a first subregion, a second subregion, and a third subregion obtained by sequentially dividing the region from a crown toward a root along a direction perpendicular to a tooth axis, and
the step of designating the portion of the region of the verification-required tooth as the region of interest comprises the step of designating, among the first to third subregions, a subregion corresponding to a lesion type of the verification-required tooth as the region of interest, wherein the lesion type of the verification-required tooth is determined using the data output by the lesion analysis model.

4. The dental lesion information visualization method of claim 3, wherein the step of designating the subregion corresponding to the lesion type of the verification-required tooth as the region of interest comprises the step of, when the lesion type of the verification-required tooth is a dental caries type, designating the first subregion as the region of interest, when the lesion type of the verification-required tooth is a periodontitis type, designating the second subregion as the region of interest, and when the lesion type of the verification-required tooth is a periapical lesion type, designating the third subregion as the region of interest.

5. The dental lesion information visualization method of claim 2, wherein the region of the verification-required tooth comprises a first subregion, a second subregion, and a third subregion obtained by sequentially dividing the region from a crown toward a root along a direction perpendicular to a tooth axis,
the first subregion comprises 1-1^{st} to 1-N^{th} subregions (where N is a natural number greater than or equal to 2) obtained by dividing the first subregion into N subregions in a direction parallel to a direction of the tooth axis,
the second subregion comprises 2-1^{st} to 2-N^{th} subregions (where N is a natural number greater than or equal to 2) obtained by dividing the second subregion into N subregions in the direction parallel to the direction of the tooth axis,
the third subregion comprises 3-1^{st} to 3-N^{th} subregions (where N is a natural number greater than or equal to 2) obtained by dividing the third subregion into N subregions in the direction parallel to the direction of the tooth axis,
and the step of designating the portion of the region of the verification-required tooth as the region of interest comprises the step of designating one of the 1-1^{st} to 1-N^{th} subregions, 2-1^{st} to 2-N^{th} subregions, and 3-1^{st} to 3-N^{th} subregions as a lesion location of the verification-required tooth using the data output by the lesion analysis model.

6. The dental lesion information visualization method of claim 1, further comprising the step of displaying the CT image of the patient's oral cavity in response to the user selection input on the indicator.

7. The dental lesion information visualization method of claim 6, wherein the step of displaying the CT image of the patient's oral cavity comprises the step of displaying the CT image with a field of view (FoV) initially set based on a location of the region of interest.

8. A dental lesion information visualization method performed by a computing system, comprising the steps of:
acquiring data of a lesion analysis model that outputs data on a type and location of a lesion included in a panoramic image of a patient's oral cavity, the lesion analysis model comprising a first artificial neural network, a second artificial neural network, and a third artificial neural network;
inputting data of the panoramic image to the first artificial neural network and performing segmentation processing on each tooth region included in the panoramic image by using data output from the first artificial neural network;
inputting data of the segmentation-processed panoramic image to the second artificial neural network and identifying a lesion included in the panoramic image for each tooth by using data output from the second artificial neural network; and
inputting an image of the tooth region with the identified lesion from the segmentation-processed panoramic image to the third artificial neural network and outputting data on the type and location of a lesion and a lesion detection confidence score by using data output from the third artificial neural network.

9. The dental lesion information visualization method of claim 8, wherein the step of performing segmentation processing on each tooth region included in the panoramic image comprises the steps of
cropping the panoramic image to display only a region of interest; and
masking tooth, crown, tooth pulp, and bone level regions in the cropped panoramic image and inputting the masked panoramic image to the first artificial neural network.

10. The dental lesion information visualization method of claim 8, wherein the step of identifying a lesion included in the panoramic image for each tooth comprises the steps of:
dividing each tooth region included in the segmentation-processed panoramic image into first through third subregions by sequentially dividing the tooth region from a crown toward a root along a direction perpendicular to a tooth axis;
inputting data of the first subregion to the second artificial neural network of a first type and identifying the presence of a dental caries lesion in the panoramic image by using data output from the second artificial neural network of a first type;
inputting data of the second subregion to the second artificial neural network of a second type and identifying the presence of a periodontal disease lesion in the panoramic image by using data output from the second artificial neural network of a second type; and
inputting data of the third subregion to the second artificial neural network of a third type and identifying the presence of a periapical inflammation lesion in the panoramic image by using data output from the second artificial neural network of a third type.

11. The dental lesion information visualization method of claim 10, wherein the step of identifying the presence of a periodontal disease lesion in the panoramic image comprises the step of identifying the presence of a periodontal disease lesion in the panoramic image by using data on an extent of depression of a bone level region relative to a crown region included in the second subregion, the data being output from the second artificial neural network of a second type.

12. The dental lesion information visualization method of claim 10, wherein the step of identifying the presence of a periapical inflammation lesion in the panoramic image comprises the step of identifying the presence of a periapical inflammation lesion in the panoramic image by using data indicating an inflammation region in the third subregion, which is adjacent to a tooth and lacks Hounsfield unit (HU) values corresponding to bone and tooth, the data being output from the second artificial neural network of a third type.

13. The dental lesion information visualization method of claim 10, wherein the step of identifying the presence of a dental caries lesion in the panoramic image comprises the step of identifying the presence of a dental caries lesion in the panoramic image by using data indicating an inflammation region with an HU value different from that of a crown region included in the first subregion, the data being output from the second artificial neural network of a first type.

14. A dental lesion information visualization system comprising:
one or more processors; and
a memory configured to load one or more instructions,
wherein the one or more processors, by executing the one or more stored instructions, perform operations including:
inputting data on a panoramic image of a patient's oral cavity to a machine-learned lesion analysis model;
determining a region of interest in the panoramic image by using data output by the lesion analysis model;
overlaying and displaying an indicator pointing to the region of interest on the panoramic image;
generating a lesion detection list using the data output by the lesion analysis model; and
displaying the lesion detection list together with the panoramic image.
